# EUROPÄISCHE PATENTSCHRIFT

(11) **EP 0 388 817 B1**
(45) Veröffentlichungstag und Bekanntmachung des Hinweises auf die Patenterteilung: **09.03.1994**
(21) Anmeldenummer: 90105026.0
(22) Anmeldetag: 16.03.1990
(51) Int. Cl.: A61K 9/127, A61K 9/107

(54) **Verwendung von Mischmicellen und Mischmicell-Lösungen von Immunmodulatoren**
Use of mixed micelles and mixed micelle solutions of immunomodulators
Utilisation de micelles mixtes et solutions de micelles mixtes d'immunomodulateurs

(30) Priorität: 21.03.1989 CH 1041/89; 12.01.1990 CH 103/90
(43) Veröffentlichungstag der Anmeldung: 26.09.1990
(73) Patentinhaber: F. HOFFMANN-LA ROCHE AG, 4002 Basel (CH)
(72) Erfinder: Hein, Wayne, Dr., CH-4125 Riehen (CH); Supersaxo, Andreas, Dr., CH-4052 Basel (CH); Steffen, Hans, Dr., CH-4410 Liestal (CH)
(74) Vertreter: Lederer, Franz, Dr.

(56) Entgegenhaltungen:
- EP-A- 0 169 571
- EP-A- 0 252 004
- EP-A- 0 280 887
- DE-A- 3 221 579
- FR-A- 2 358 161

## Beschreibung

Die vorliegende Erfindung betrifft die Verwendung von Mischmicellen als Träger für parenterale Verabreichung von pharmazeutischen Wirkstoffen, sowie Mischmicell-Lösungen, die bestimmte pharmazeutische Wirkstoffe enthalten.

Es ist bekannt, Mischmicellen als Vehikel für pharmazeutische Wirkstoffe zu verwenden. Die DE-PS 27 30 570 beschreibt Mischmicell-Lösungen zur parenteralen Verabreichung von fettlöslichen, d.h. in Wasser nicht oder schwer löslichen Wirkstoffen, insbesondere Benzodiazepinen und Vitamin K. Die EP-A2-133 258 beschreibt Mischmicell-Lösungen zur parenteralen Verabreichung von Vitamin E. Weiterhin sind Mischmicellen zur Herstellung von wässrigen Proteinlösungen vorgeschlagen worden (EP-A1-252004). Bei der Verwendung von Mischmicellen zur Solubilisierung von nicht-steroidalen Entzündungshemmern gemäss der EP-A1-280 887 lag die Aufgabe zugrunde, die Verträglichkeit der Wirkstoffe bei Injektionen zu verbessern.

Bei der parenteralen, insbesondere der interstitiellen, z.B. der intramuskulären, subkutanen oder intradermalen Verabreichung pharmazeutischer Wirkstoffe werden solche mit einem Molgewicht <1000 kaum lymphatisch aufgenommen, während Wirkstoffe, deren Molgewicht etwa 16 000 oder mehr beträgt, zu einem wesentlichen Teil vom lymphatischen System aufgenommen werden.

Es kann jedoch erwünscht sein, auch niedermolekulare Wirkstoffe, die normalerweise nicht in wesentlichem Umfang vom lymphatischen System transportiert werden, in dieses einzubringen und so an den Zielort zu transportieren. Dies ist insbesondere bei das Immunsystem beeinflussenden Wirkstoffen, wie Immunsupressiva und Immunstimulantien der Fall. Ueberraschend wurde gefunden, dass Mischmicellen bei interstitieller Applikation in Liposomen umgewandelt werden, die vom lymphatischen System aufgenommen und transportiert werden. Bei Vorliegen bestimmter Voraussetzungen werden dabei in Mischmicellen enthaltene Wirkstoffe von den im Organismus sich bildenden Liposomen aufgenommen. Ob im jeweiligen Fall solche Voraussetzungen gegeben sind, kann experimentell durch die Bestimmung des Verteilungskoeffizienten des Wirkstoff in Octanol/Wasser ermittelt werden. Wirkstoffe, deren Verteilungskoeffizient im System Octanol/Wasser grösser als etwa 10⁴, vorzugsweise grösser als etwa 10⁶ ist, erfüllen die Voraussetzung, d.h., werden von den sich bildenden Liposomen aufgenommen.

Fettlösliche Vitamine, z.B. die Vitamine A, E und K haben einen Verteilungskoeffizienten von >10⁶ in Octanol/Wasser. Wie oben erwähnt, waren Mischmicellösungen solcher Vitamine bekannt. Nicht bekannt war jedoch, dass diese Vitamine nach parenteraler, insbesondere interstitieller Verabreichung als Mischmicell-Lösungen in Form von Liposomen in die Lymphbahn gelangen.

Gegenstand der Erfindung ist daher die Verwendung von Mischmicellen als Wirkstoffträger für die Herstellung parenteral zu verabreichender therapeuticher Zubereitungen niedermolekularer Wirkstoffe mit einem Verteilungskoeffizient in Octanol/Wasser von grösser als etwa 10⁴, wobei fettlösliche Vitamine ausgenommen sind.

Als niedermolekulare Wirkstoffe werden im vorliegenden Zusammenhang Wirkstoffe mit einem Molgewicht unter 10 000 verstanden. Vorzugsweise haben die niedermolekularen Wirkstoffe ein Molgewicht unter 5000.

Beispiele von Wirkstoffen, bei deren parenteraler, insbesondere interstitieller therapeutischer Verabreichung erfindungsgemäss die Verwendung von Mischmicellen als Träger in Betracht kommt, sind Immunmodulatoren, z.B. Immunsupressiva wie (all-E)-3,7-Dimethyl-9-(2-trifluormethyl)-6-(nonyloxy)phenyl -2,4,6,8-nonatetraensäure und verwandte Verbindungen, die in der EP-A1-169 571 beschrieben sind; Cyclosporin oder FK 506 (Immunology Today 10, 6-9 (1989)); sowie lipophile Derivate davon, z.B. C₁₋₂₀-Alkanoyloxyderivate; Immunstimulantien, wie N-Acetylmuramyl-L-alanyl-D-isoglutamyl-L-alanyl -2-(1′,2′-dipalmitoyl)phosphatidyl-äthanolamin (Cancer Immunol. Immunother. (1986) 22: 191-196); Peptid- und Oligosaccharidantigene oder lipophile Derivate davon, z.B. Peptid-Fettsäureesterkonjugate (EP-A2-290 246); Kontrastmittel zur Lymphszintigraphie; Cytostatika, z.B. cis-Pt-Komplexe wie cis-bis-N-Decylaminodiacetato-1,2-diaminocyclohexan-platin; oder Anthracycline wie Doxorubicin oder Methotrexat bzw. lipophile Derivate davon wie die oben erwähnten Fettsäureester, oder lipophile Derivative von 5-Fluor-2′-deoxyuridin, z.B. Palmitoate (Microencapsulation 1988, Vol 5, No. 1, p. 1-11) und Phosphate (Japan. Kokai 1091 195); lipophile Derivate von 1-β-D-Arabinofuranosylcytosin (Ara-C), wie Oleate und Palmitate (Int. J. Cancer 37, 149-154 [1986]) und Cholesteryloxycarbonylglycyl-Ara-C (Chem. Pharm. Bull Vol. 36 (1988) 4060 et seq.); Phosphatidylinosit und Lysophospholipide (DE-OS 3008082); und Anti-infektiva wie Amphotericin B, Nystatin, Gentamycin, Chloroquin, Penicilline, wie Ampicillin; und Tetracycline bzw. lipophile Derivate davon, wie die oben erwähnten Fettsäureester.

Die vorstehend genannten Wirkstoffe können mit an sich bekannten Technologien in Mischmicellen inkorporiert werden, beisielsweise wie in der DE-PS 27 30 570 beschrieben. Mischmicell-Lösungen sind im Vergleich zu liposomalen Lösungen einfacher herzustellen und weisen eine bessere Stabilität auf. Die erfindungsgemäss herstellbaren Mischmicell-Lösungen vereinigen daher die Vorteile der Stabilität von Mischmicell-Lösungen mit der verbesserten Wirksamkeit lymphatisch und/oder liposomal applizierter Wirkstoffe.

Im Rahmen der vorliegenden Erfindung werden unter Mischmicellen bzw. Mischmicell-Lösungen wässrige, homogene Lösungen von Gallensäurederivaten, insbesondere Alkalisalzen davon und Lipiden verstanden. Beispiele von Gallensäuresalzen sind Na-Cholat, Glycocholat, Taurocholat, Deoxycholat, Glyco- und Taurodeoxycholat, Chenodeoxycholat; und Glyco- und Taurochenodeoxycholat. Beispiele von Lipiden sind natürliche, semisynthetische und vollsynthetische Lipide, insbesondere Phosphatidylcholine, Glycerinäther-Phosphatide, Phosphatidyläthanolamin, Phosphatidylinositol, Phosphatidylserine, Sphingomyelin, Plasmalogene, Cardiolipin, Sulfatide und Monoglyceride. Die Mischmicellen können als zusätzliche Komponenten Cholesterin (bis etwa 30 Mol-% bezogen auf den Lipidanteil) und Lipide mit negativ oder positiv geladenen Gruppen enthalten, wie Phosphatidsäuren oder Stearylamin (bis etwa 10 Mol-% bezogen auf den Lipidanteil)
Die Mischmicell-Lösungen können durch blosses Zusammenmischen der einzelnen Bestandteile hergestellt werden. Es ist jedoch von Vorteil, den Lipidbestandteil, den Micellbildner und den bzw. die in Wasser schwer oder unlöslichen Wirkstoff(e) in einem organischen Lösungsmittel zu lösen, darauf das organische Lösungsmittel einzudampfen und hierauf das Wasser, die isotonisierenden Zusätze und gegebenenfalls die weiteren Ingredientien zuzugeben, wobei in der Regel die isotonisierenden Zusätze und zumeist auch die allfälligen weiteren Ingredientien vor der Zugsbe zum erwähnten Eindampfrückstand mit dem Wasser vermischt werden. Als organische Lösungsmittel kommen solche in Betracht, in denen die zu lösenden Komponenten hinreichend löslich sind, wie z.B. niedere Alkanole, insbesondere Aethanol.

Ein besonderes bevorzugtes Verfahren besteht indessen darin, dass man ungefähr einen molaren Teil Micellbildner, ungefähr einen molaren Teil Lipidbestandteil und ungefähr 0,3 bis einen molaren Teil Wasser, gegebenenfalls in Gegenwart von bis zu 2% eines organischen Lösungsmittels, wie Aethanol, sowie den bzw. die in Wasser schwer oder unlöslichen Wirkstoff(e) intensiv rührt, bis das Gemisch homogen erscheint, worauf das Wasser, die isotonisierenden Zusätze und gegebenenfalls die weiteren Ingredientien zugesetzt werden, bis die gewünschte Verdünnung bzw. Konzentration erreicht ist. Es ist aber auch möglich, das obige Verfahren ohne Wirkstoff durchzuführen und erst am Schluss den oder die Wirkstoff(e) in der micellaren Lösung zu solubilisieren.

Das Verhältnis zwischen dem Lipidbestandteil und dem Micellbildner liegt in der Grössenordnung von 0,1:1 bis 2:1. Bevorzugt sind Mischungsverhältnisse von 0,1:1 bis 0,8:1 und 1,5:1 bis 2:1. Ganz besonders bevorzugt ist das Mischungsverhältnis 0,8:1 bis 1,5:1.

Der Anteil von Lipidbestandteil plus Micellbildner in der Injektionslösung kann über weite Grenzen variieren und z.B. 2-200 mg/ml Injektionslösung betragen.

Der Anteil des Pharmakons in der Mischmicell-Lösung kann ebenfalls über weite Grenzen variieren und z.B. 0,1-100 mg/ml Injektionslösung betragen.

Zur Verhinderung von Oxidationsreaktionen des Wirkstoffs und der Trägermaterialien können Antioxidantien, wie Tocopherole, Ascorbinpalmitat, Natriumascorbat, Natriumhydrogensulfit, Natriumpyrosulfit oder Natriumsulfit zugesetzt werden.

Weitere Hilfsstoffe zur pH-Einstellung, z.B. Phosphat-, Citrat- oder Tris-Puffer; zur Isotonisierung, z.B. Natriumchlorid, Mannitol, Sorbitol oder Glucose und zur Konservierung, z.B. Methyl- und Propyl-p-Hydroxybenzoesäure, Benzylalkohol oder Phenol können ebenfalls zugegeben werden.

Falls erwünscht, lassen sich die Mischmicell-Lösungen mit Hilfe konventioneller Lyophilisierungsverfahren in Trockenpräparate überführen.

Die nachstehenden Beispiele sollen die Erfindung weiter erläutern.

### Beispiel 1

### Herstellung von Mischmicell-Lösungen:

### Wirkstoff (all-E)-3,7-Dimethyl-9-(2-trifluormethyl)-6-(nonyloxy)phenyl -2,4,6,8-nonatetraensäure (A)

30,8 mg Sojalecithin (Epikuron 200, Lucas Meyer, Hamburg, Deutschland), 21,40 mg Natriumglycocholat und 0,4 mg (all-E)-3,7-Dimethyl-9-(2-trifluormethyl) -6-(nonyloxy)phenyl-2,4,6,8-nonatetraensäure werden in einem Rundkolben in 5 ml Chloroform/Methanol (1:1, Vol/Vol%) gelöst. Der nach Abdampfen des organischen Lösungsmittel (Rotationsverdampfer, 40°C) resultierende Film wird in 1 ml 3,8%-iger Mannitlösung (Gew/Vol%) dispergiert. Die erhaltenen Mischzellen werden mit 1N HCl auf pH 6,0 ± 0,1 eingestellt, sterilisiert, in Vialen abgefüllt und lyophilisiert. Zur Verhinderung von Isomerisierungsreaktionen des Wirkstoffs sowie Oxidationsreaktionen des Wirkstoffs und der Trägermaterialien, insbesondere der Phospholipide, werden die Mischmicellen unter Ausschluss von Licht und in Inertgasatmosphäre, z.B. unter Stickstoff, hergestellt.

### Beispiel 2

### Wirkstoff 3′,5′-Di-O-palmitoyl-5-fluor-2′-deoxuridin (B)

30,8 g Sojalecithin (Epikuron 200, Lucas Meyer, Hamburg, Deutschland), 21,67 mg Natriumglycocholat und 1,0 mg 3′,5′-Di-O-palmitoyl-5-fluor-2′-deoxyuridin werden in einem Rundkolben in 5 ml Chloroform/Methanol (1:1. Vol/Vol%) gelöst. Der nach Abdampfen des organischen Lösungsmittels (Rotationsverdampfer, 40°C) resultierende Film wird in 1 ml 3,8%-iger Mannitlösung dispergiert. Die erhaltenen Mischzellen werden mit 1N HCl auf pH 6,0 ± 0,1 eingestellt, sterilisiert und in Ampullen abgefüllt. Zur Verhinderung von Oxidationsreaktionen des Wirkstoffs sowie der Trägermaterialien, insbesondere der Phospholipide, wird die Herstellung der Mischzellen in Inertgasatmosphäre, z.B. unter Stickstoff durchgeführt.

Die Mischmicell-Lösungen wurden Versuchstieren (Schafe) mittels einer in der EP-A-272530 beschriebenen Vorrichtung intradermal am rechten Hinterfuss verabreicht. Blut wurde durch einen Jugularvenen-Katheter und Lymphflüssigkeit durch eine Kanüle in der ableitenden Lymphbahn am rechten poplitealen Lymphknoten) entnommen (Pharm. Res. Vol. 5, 472-476 (1988)).

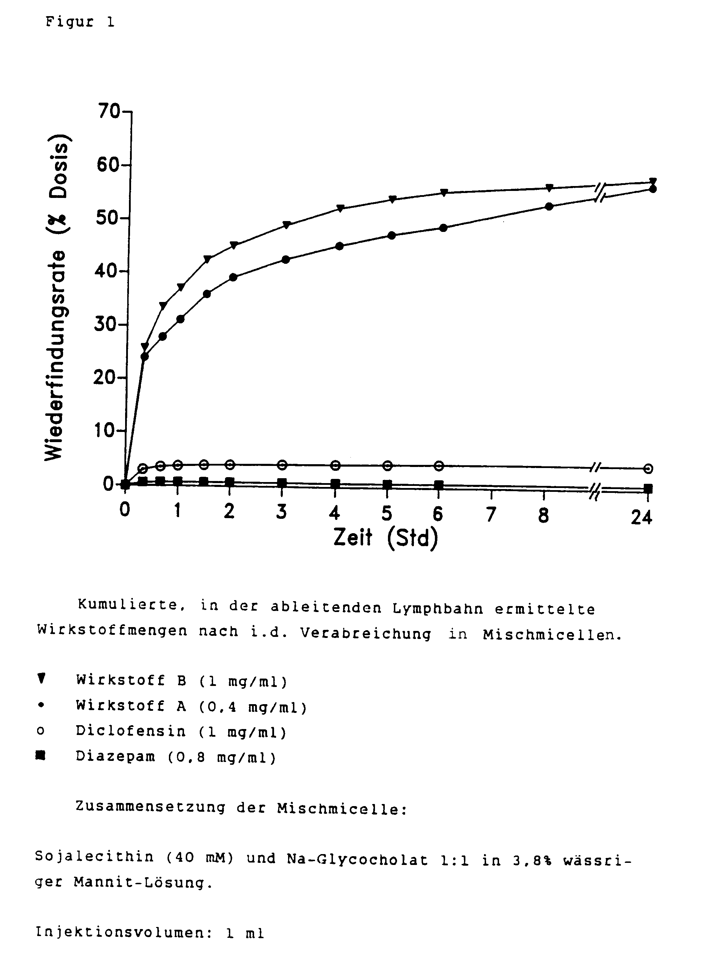

Figur 1 zeigt den in der Lymphe gefundenen Anteil der verabreichten Dosis. Zum Vergleich sind die Werte angeführt. die mit zwei niedermolekularen Wirkstoffen, deren Verteilungskoeffizient in Octanol/Wasser <10⁴ ist, erhalten wurden.

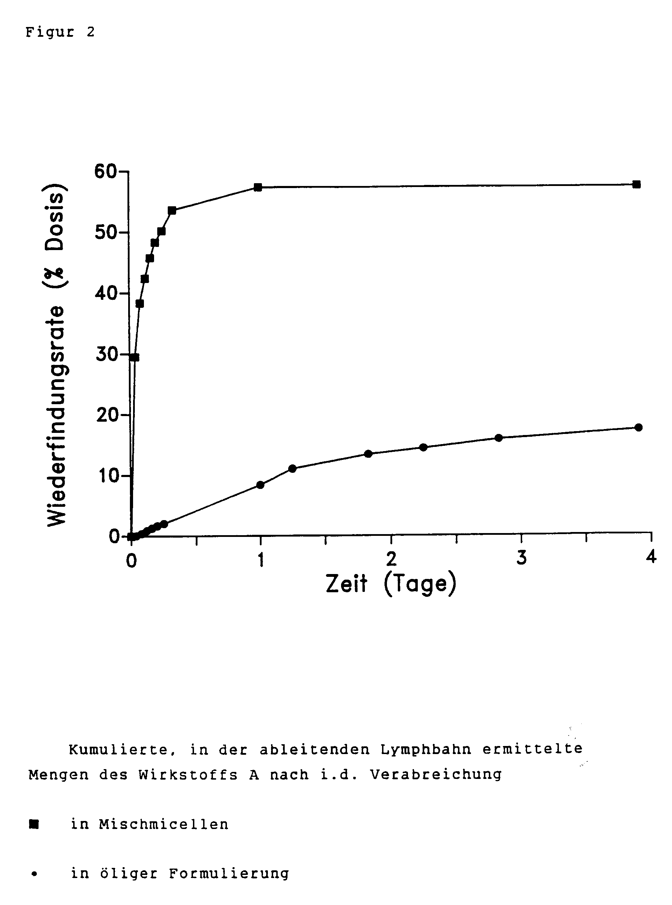

Figur 2 zeigt die in der Lymphe gefundene Mengen des Wirkstoffs A nach Verabreichung in Mischmicellen und als ölige Formulierung.

## Patentansprüche (Patentansprüche für folgende(n) Vertragsstaat(en): AT, BE, CH, DE, DK, FR, GB, IT, LI, LU, NL, SE)

1. Verwendung von Mischmicellen als Wirkstoffträger für die Herstellung parenteral zu verabreichender therapeutischer Zubereitungen niedermolekularer Wirkstoffe mit einem Verteilungskoeffizient in Octanol/Wasser von grösser als 10⁴, ausgenommen fettlösliche Vitamine.

2. Verwendung nach Anspruch 1 für Wirkstoffe mit einem Verteilungskoeffizienten in Octanol/Wasser grösser als 10⁶ und einem Molgewicht unter 5000.

3. Verwendung nach Anspruch 1 oder 2, wobei der Wirkstoff ein Immunmodulator, ein Cytostatikum, ein Antiinfektivum oder Kontrastmittel ist.

4. Verwendung nach den Ansprüchen 1-3 zur interstitiellen Applikation.

5. Verwendung nach Anspruch 4 zur intramuskulären, subkutanen oder intradermalen Applikation.

6. Mischmicell-Lösung, enthaltend einen niedermolekularen Immunmodulator mit einem Verteilungskoeffizient in Octanol/Wasser von grösser als 10⁴.

7. Mischmicell-Lösung nach Anspruch 6, wobei der Immunmodulator (all-E)-3,7-Dimethyl-9-(2-trifluormethyl)-6-(nonyloxy)phenyl-2,4,6,8-nonatetraensäure ist.

## Patentansprüche (Patentansprüche für folgende(n) Vertragsstaat(en): ES, GR)

1. Verwendung von Mischmicellen als Wirkstoffträger für die Herstellung parenteral zu verabreichender therapeutischer Zubereitungen niedermolekularer Wirkstoffe mit einem Verteilungskoeffizient in Octanol/Wasser von grösser als 10⁴, ausgenommen fettlösliche Vitamine.

2. Verwendung nach Anspruch 1 für Wirkstoffe mit einem Verteilungskoeffizienten in Octanol/Wasser grösser als 10⁶ und einem Molgewicht unter 5000.

3. Verwendung nach Anspruch 1 oder 2, wobei der Wirkstoff ein Immunmodulator, ein Cytostatikum, ein Antiinfektivum oder Kontrastmittel ist.

4. Verwendung nach den Ansprüchen 1-3 zur interstitiellen Applikation.

5. Verwendung nach Anspruch 4 zur intramuskulären, subkutanen oder intradermalen Applikation.

6. Verfahren zur Herstellung von Mischmicell-Lösungen, enthaltend Micellbildner für Mischmicellen, einen niedermolekularen Immunomodulator mit einem Verteilungskoeffizient in Octanol/Wasser von grösser als 10⁴, Wasser und gegebenenfalls pharmazeutische Hilfsstoffe, dadurch gekennzeichnet, dass man die einzelnen Bestandteile miteinander vermischt.

7. Verfahren nach Anspruch 6, dadruch gekennzeichnet, das der Immunmodulator (all-E)-3,7-Dimethyl-9-(2-trifluormethyl)-6-(nonyloxy)phenyl-2,4,6,8-nonatetraensäure ist.

## Claims (Claims for the following Contracting State(s): AT, BE, CH, DE, DK, FR, GB, IT, LI, LU, NL, SE)

1. The use of mixed micelles as active substance carriers for the manufacture of parenterally administerable therapeutic compositions of low-molecular active substances having a distribution coefficient in octanol/water of greater than about 10⁴, with fat-soluble vitamins being excluded.

2. The use according to claim 1 for active substances having a distribution coefficient in octanol/water of greater than 10⁶ and a molecular weight below 5000.

3. The use according to claim 1 or 2, in which the active substance is an immunomodulator, a cytostatic an antiinfective or contrast agent.

4. The use according to claims 1-3 for interstitial application.

5. The use according to claim 4 for intramuscular, subcutaneous or intradermal application.

6. A mixed micelle solution containing a low molecular immunomodulator having a distribution coeffiecient in octanol/water of greater than 10⁴.

7. A mixed micelle solution according to claim 6, in which the immunomodulator is (all-E)-3,7-dimethyl-9-(2-trifluoromethyl)-6-(nonyloxy)phenyl-2,4,6,8-nonatetraenoic acid.

## Claims (Claims for the following Contracting State(s): ES, GR)

1. The use of mixed micelles as active substance carriers for the manufacture of parenterally administerable therapeutic compositions of low-molecular active substances having a distribution coefficient in octanol/water of greater than about 10⁴, with fat-soluble vitamins being excluded.

2. The use according to claim 1 for active substances having a distribution coefficient in octanol/water of greater than 10⁶ and a molecular weight below 5000.

3. The use according to claim 1 or 2,in which the active substance is an immunomodulator, a cytostatic, an antiinfective or contrast agent.

4. The use according to claims 1-3 for interstitial application.

5. The use according to claim 4 for intramuscular, subcutaneous or intradermal application.

6. A process for the manufacture of mixed micelle solutions containing a micelle former for mixed micelles, a low molecular immunomodulator having a distribution coefficient in octanol/water of greater than 10⁴, water, and, if desired, pharmaceutical adjuvants, characterized in that the individual ingredients are mixed together.

7. A process according to claim 6, characterized in that immunomodulator is (all-E)-3,7-dimethyl-9-(2-trifluoromethyl)-6-(nonyloxy)phenyl-2,4,6,8-nonatetraenoic acid.

## Revendications (Revendications pour l'(les) Etat(s) contractant(s) suivant(s): AT, BE, CH, DE, DK, FR, GB, IT, LI, LU, NL, SE)

1. Utilisation de micelles mélangées en tant que véhicules de substances actives pour la préparation de compositions thérapeutiques pour l'administration parentérale contenant des substances actives à bas poids moléculaire ayant un coefficient de répartition supérieur à 10⁴ dans le mélange octanol/eau, à l'exception de vitamines liposolubles.

2. Utilisation selon revendication 1, pour des substances actives ayant un coefficient de répartition supérieur à 10⁶ dans le mélange octanol/eau et un poids moléculaire inférieur à 5000.

3. Utilisation selon revendication 1 ou 2, dans laquelle la substance active est immunomodulateur, un cytostatique, un agent anti-infectieux ou un agent de contraste.

4. Utilisation selon les revendications 1 à 3, pour l'administration interstitielle.

5. Utilisation selon revendication 4, pour l'administration intramusculaire, sous-cutanée ou intradermique.

6. Solution à micelles mélangées, contenant un immunomodulateur à bas poids moléculaire ayant un coefficient de répartition supérieur à 10⁴ dans le mélange octanol/eau.

7. Solution à micelles mélangées selon revendication 6, dans laquelle l'immunomodulateur est l'acide (Tout-E)-3,7-diméthyl-9-(2-trifluorométhyl)-6-(nonyloxy)-phényl-2,4,6,8-nonatétraénoïque.

## Revendications (Revendications pour l'(les) Etat(s) contractant(s) suivant(s): ES, GR)

1. Utilisation de micelles mélangées en tant que véhicules de substances actives pour la préparation de compositions thérapeutiques pour l'administration parentérale contenant des substances actives à bas poids moléculaire ayant un coefficient de répartition supérieur à 10⁴ dans le mélange octanol/eau, à l'exception de vitamines liposolubles.

2. Utilisation selon revendication 1, pour des substances actives ayant un coefficient de répartition supérieur à 10⁶ dans le mélange octanol/eau et un poids moléculaire inférieur à 5000.

3. Utilisation selon revendication 1 ou 2, dans laquelle la substance active est immunomodulateur, un cytostatique, un agent anti-infectieux ou un agent de contraste.

4. Utilisation selon les revendications 1 à 3, pour l'administration interstitielle.

5. Utilisation selon revendication 4, pour l'administration intramusculaire, sous-cutanée ou intradermique.

6. Procédé de préparation de solutions de micelles mélangées contenant des formateurs de micelles pour micelles mélangées, un immunomodulateur à bas poids moléculaire ayant un coefficient de répartition supérieur à 10⁴ dans le mélange octanol/eau et le cas échéant des produits auxiliaires pharmaceutiques, caractérisé en ce que l'on mélange les constituants individuels entre eux.

7. Procédé selon revendication 6, caractérisé en ce que l'immunomodulateur est l'acide (tout-E)-3,7-diméthyl-9-(2-trifluorométhyl)-6-(nonyloxy)-phényl-2,4,6,8-nonatétraénoïque.
